(19) Europäisches Patentamt
European Patent Office
Office européen des brevets

(11) Veröffentlichungsnummer: **0 383 023 B1**

# (12) EUROPÄISCHE PATENTSCHRIFT

(45) Veröffentlichungstag der Patentschrift: **13.04.94**

(51) Int. Cl.[5]: **C07H 15/04**, C08F 20/36, C08F 299/02

(21) Anmeldenummer: **90100738.5**

(22) Anmeldetag: **15.01.90**

Die Akte enthält technische Angaben, die nach dem Eingang der Anmeldung eingereicht wurden und die nicht in dieser Patentschrift enthalten sind.

## (54) Polymerisierbare N'-Polyhydroxy-N'-alkylharnstoffderivate, Verfahren zu ihrer Herstellung, deren Verwendung und daraus erhältliche Homo- oder Copolymeren.

(30) Priorität: **13.02.89 DE 3904246**

(43) Veröffentlichungstag der Anmeldung:
**22.08.90 Patentblatt 90/34**

(45) Bekanntmachung des Hinweises auf die Patenterteilung:
**13.04.94 Patentblatt 94/15**

(84) Benannte Vertragsstaaten:
**AT BE CH DE DK ES FR GB GR IT LI LU NL SE**

(56) Entgegenhaltungen:
**EP-A- 0 220 676**
**EP-A- 0 243 890**
**EP-A- 0 255 033**
**DE-A- 1 125 905**
**DE-A- 1 816 279**
**US-A- 2 834 775**
**US-A- 2 922 784**
**US-A- 4 320 221**

**DIE MAKROMOLEKULARE CHEMIE, RAPID COMMUNICATIONS, Band 10, Nr. 12, Dezember 1989, Seiten 629-636, Hüthig & Wepf Verlag, Basel, CH; J. KLEIN et al.: "Poly(vinylsaccharide)s, 6a. Synthesis and characterisation of new poly(vinylsaccharide)s of the urea type"**

(73) Patentinhaber: **SÜDZUCKER AKTIENGESELLSCHAFT MANNHEIM/OCHSENFURT**
**Maximilianstrasse 10**
**D-68165 Mannheim(DE)**

(72) Erfinder: **Begli, Ali Reza Haji, Dr. rer. nat.-Dipl.-Chem.**
**Rebenring 62**
**D-3300 Braunschweig(DE)**
Erfinder: **Klein, Joachim, Prof.-Dr.rer.nat.Dipl.-Chem.**
**Hühnerkamp 21**
**D-3300 Braunschweig(DE)**
Erfinder: **Kowalczyk, Jörg, Dipl.-Chem.**
**Bauslebenring 14**
**D-3307 Kneitlingen(DE)**
Erfinder: **Kunz, Markwart, Dr.rer.nat. Dipl.-Chem.**
**Am Kötherberg 6**
**D-3300 Braunschweig(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 383 023 B1

**Research Disclosure Nr.208, 08/1981, S.309-310, Veröffentlichungsnr. 20816**

**Die Makromolukulare Chemie, Macromolecular Chemistry and Physics, 188(6), 1217-1232 (1987)**

**Journal of polymer science 50, 127-132 (1961)**

**A. HAJI BEGLI : "Synthese und Charakterisierung neuer Polyvinylsaccharide mit Amid bzw. Harnstoff-Verknüpfung", Dissertation, TU Braunschweig, 1988**

**S. ENGELKE : "Synthese und Charakterisierung von ionischen und nichtionischen Polyvinylsaccharide mit Dissacharidseitengruppen", Disseration, TU Braunschweig, 1989**

(74) Vertreter: **Gleiss, Alf-Olav, Dipl.-Ing.**
**Gleiss & Grosse**
**Patentanwaltskanzlei**
**Maybachstrasse 6A**
**D-70469 Stuttgart (DE)**

## Beschreibung

Die vorliegende Erfindung betrifft die im Anspruch 1 aufgeführten neuen Verbindungen entsprechend den Formeln I oder II, die Herstellung dieser Monomeren aus reduzierenden Mono- und Oligosacchariden, Alkylaminen und Vinyl- bzw. Acrylisocyanaten, sowie die Verwendung der derart hergestellten Monomeren zur Darstellung von oberflächenaktiven Polymeren unterschiedlichen HLB-Wertes und schließlich die aus diesen Monomeren durch Redox-Initiation erhältlichen Homopolymere oder Copolymere.

Die Konstitution der beanspruchten monomeren Verbindungen gemäß den Formeln I und II ist durch $^{13}C$-NMR-Spektroskopie gesichert.

Die Herstellung von N-Alkylaminopolyolen aus reduzierenden Kohlenhydraten und N-Alkylaminien ist in der Literatur erwähnt (US-PS 4,021,539, DE-OS 18 16 279, DE-A 15 93 894).

Aus der Dissertation A.H. Begli "Synthese und Charakterisierung neuer Polyvinylsaccharide mit Amid bzw. Harnstoff-Verknüpfung", Braunschweig 1988, ist ferner bekannt, das 1-Aminosaccharide mit Acrylsäure- oder Methacrylsäureanhydrid bzw. -chlorid zum entsprechenden Acrylamidosaccharid kondensiert werden können. Ferner ist beschrieben, die 1-Aminosaccharide mit Isocyanat in die entsprechenden N-Vinyl-N'-saccharidharnstoffe zu überführen. Überraschenderweise lassen sich die Vinylharnstoffsaccharide nicht zu hohen Kettenlängen polymerisieren. Acrylamidosaccharide und N-Acryloylethyl-N'saccharidharnstoffe lassen sich dagegen zu längeren Polyacrylatketten kondensieren. Die Polyharnstoffderivate zeigen dabei gegenüber den Amiden eine bessere Polymerisationsausbeute und höhere Löslichkeit, haben aber Nachteil, daß die Esterbindung leicht hydrolytisch gespalten wird. N-Alkylacrylamidosaccharide mit Alkylgruppen mit mehr als 4 C-Atomen lassen sich ebenfalls nicht mehr polymerisieren, was eventuell auf die zusätzliche sterische Hinderung zurückzuführen ist.

Es stellte sich daher die Aufgabe, polymerisierbare Derivate der N-Alkylaminosaccharide zu finden, welche beständige Polymere liefern, welche gut wasserlöslich sind und als oberflächenaktive Verbindungen eingesetzt werden können. Diese Aufgabe wird durch die Merkmale des Hauptanspruchs gelöst und durch die der Unteransprüche gefördert.

Die Herstellung der erfindungsgemäßen Monomeren entsprechend den Formeln I oder II erfolgt aus N-Alkylaminopolyolen durch selektive Addition von substituierten Isocyanaten in Gegenwart von Wasser und/oder Alkoholen oder einem inerten aprotischen Lösungsmittel, wie beispielsweise Dioxan oder Tetrahydrofuran, besonders bei Temperaturen zwischen 0 und 25 °C.

Als Aminopolyole können zum Beispiel eingesetzt werden: Maltamin, Isomaltamin, Glucamin und deren Mischungen. Innerhalb der Alkylreste mit 4 bis 21 C-Atomen seien genannt: Hexyl-, Octyl-, Decyl-. Als Reaktionskomponenten mit den N-Alkylaminopolyolen dienen außer Vinylisocyanat in der Gruppe der acrylierten Isocyanate besonders Isocyanatoethylacrylat und Isocyanatoethylmethacrylat.

Die Bezeichnung für die erfindungsgemäßen Monomeren gemäß den Formeln I und II ist je nach dem verwendeten Isocyanat unterschiedlich:

- Bei Verwendung von Vinylisocyanat[1)]als geeignet substituiertes Isocyanat empfiehlt sich als Bezeichnung N-Vinyl-N',N'-alkyldesoxysaccharidalkohol-harnstoff[2)].
- Bei Verwendung von Isocyanatoethylmethacrylat[3)]als Reaktionspartner empfiehlt sich die Bezeichnung N-Ethylmethacrylato-N',N'-alkyldesoxysaccharidalkohol-harnstoff[4)].

Die erfindungsgemäß hergestellten Monomeren unterscheiden sich in ihren HLB-Werten (Hydrophilic/lipophilic balance, "Das Atlas HLB System, Atlas Chemie GmbH, Essen, 1968") entsprechend der Wahl der verwendeten Alkylamine und der reduzierenden Kohlenhydrate. In entsprechender Weise ist die Oberflächenspannung von wäßrigen Lösungen der erfindungsgemäßen Monomeren, aber auch der daraus hergestellten Polymeren, unterschiedlich (Beispiele siehe Tabelle 1). Auch in anderen polymertypischen Charakterisierungsdaten sind die Produkte verschieden (Beispiele siehe Tabelle 2).

Die Homo- oder Copolymerisation der erfindungsgemäßen Monomeren erfolgt bevorzugt durch Initiation mit Peroxiden oder Azo-Derivaten in wässerigem Medium bei Temperaturen zwischen 0 und 100 °C.

[1)] Als Kurzform wird im folgenden für Vinylisocyanat VIC benutzt.

[2)] Als Kurzform empfiehlt sich VIC-alkylmonomer.

[3)] Als Kurzform wird im folgenden für Isocyanatoethyl

methacrylat IEM benutzt. [4)] Als Kurzform empfiehlt sich IEM-alkylmonomer.

Tabelle 1

| Grenzflächenspannungen von wäßrigen Lösungen von einigen erfindungsgemäßen Monomeren und Polymeren, sowie von einigen Ausgangsverbindungen bei 25 °C | | |
|---|---|---|
| Substanz | Molekulargewicht (g/mol) | Grenzflächenspannung 0,1%-ige Lsg (mN/m) |
| Wasser* | 18 | 72 |
| Maltose | 342 | 72 |
| 1-Aminomaltit | 343 | 72 |
| N-Butylaminomaltit | 397 | 71 |
| IEM-butyl-aminomaltitmonomer | 554 | 64 |
| Butyl-aminomaltitpolymer | > $10^6$ | 66 |
| N-Hexylaminomaltit | 425 | 56 |
| IEM-hexylaminomaltitmonomer | 582 | 51 |
| Hexyl-aminomaltitpolymer | > $10^6$ | 55 |
| N-Hexylaminoisomalt | 425 | 54 |
| IEM-hexylaminoisomaltmonomer | 582 | 51 |
| Hexyl-aminoisomaltpolymer | > $10^6$ | 52 |
| N-octylaminomaltit | 453 | 50 |
| IEM-octyl-aminomaltitmonomer | 610 | 46 |
| Octyl-aminomaltitmonomer | < $10^6$ | 47 |
| PAAm[1)] | > $10^6$ | 73 |
| CAOl[2)] | > $10^6$ | 53 |
| N-Decylaminomaltit | 481 | 37 |
| IEM-decyl-aminomaltitmonomer | 636 | 36 |
| Decyl-aminomaltitmonomer | 0,8 x $10^6$ | 35 |
| VIC-hexyl-monomer | 496 | 55 |
| PVP[3)] | > $10^5$ | 70 |
| VIC-hexyl/VP-polymer[4)] | > $10^5$ | 58 |

* Lösungsmittel
1) Polyacrylamid
2) Polyacrylamidcopolymer mit IEM-Octylaminomaltit
3) Polyvinylpyrrolidon
4) Vinylpyrrolidoncopolymer

Tabelle 2

Charakterisierungsdaten von einigen synthetisierten wasserlöslichen Polymeren

| Pol.Nr. | $M_w$ [g/mol] | $P_w$ | [η] [ml/g] | dn/dc [ml/g] |
|---|---|---|---|---|
| Poly-N-ethylmethacrylato-N',N'-butyl,1-desoxy-maltit-harnstoffe | | | | |
| P 101 | $1.04\cdot10^6$ | 1888 | 37.3 | 0.156 |
| P 102 | $1.78\cdot10^6$ | 3231 | 60.8 | 0.158 |
| P 103 | $3.30\cdot10^6$ | 5976 | 83.5 | 0.157 |
| P 104 | $4.07\cdot10^6$ | 7500 | 98.5 | 0.157 |
| P 105 | $4.47\cdot10^6$ | 8097 | 107.0 | 0.156 |
| P 106 | $9.47\cdot10^6$ | 17172 | 151.7 | 0.157 |
| Poly-N-ethylmethacrylato-N',N'-hexyl,1-desoxy-maltit-harnstoffe | | | | |
| P 201 | $0.79\cdot10^6$ | 1360 | 22.2 | 0.145 |
| P 202 | $2.90\cdot10^6$ | 4991 | 42.1 | 0.146 |
| P 203 | $3.91\cdot10^6$ | 6729 | 56.0 | 0.147 |
| P 204 | $4.87\cdot10^6$ | 8382 | 62.0 | 0.146 |
| P 205 | $18.36\cdot10^6$ | 31546 | 136.0 | 0.146 |
| Poly-N-ethylmethacrylato-N',N'-octyl,1-desoxy-maltit-harnstoffe | | | | |
| P 301 | $0.77\cdot10^6$ | 1264 | 17.4 | 0.112 |
| P 302 | $0.83\cdot10^6$ | 1362 | 21.5 | 0.113 |
| Poly-N-ethylmethacrylato-N',N'-decyl,1-desoxy-maltit-harnstoff | | | | |
| P 401 | $0.73\cdot10^6$ | 998 | 14.3 | 0.108 |
| Poly-N-ethylmethacrylato-N',N'-hexyl,2-desoxy-isomalt-harnstoff | | | | |
| P 501 | $1.2\cdot10^6$ | 2058 | 26.1 | 0.145 |

Überraschenderweise unterscheiden sich die Oberflächenspannungen wässeriger Lösungen der N-Alkylaminopolyole, aus ihnen hergestellte Monomere, Homopolymere und selbst Copolymere kaum (siehe Tabelle 1). Mit zunehmender Alkylkette bei gleichbleibendem hydrophilen Teil (Kohlenhydrat) sinkt die Oberflächenspannung wäßriger Lösungen erwartungsgemäß.

Abb. 1

Oberflächenspannung 0,1%-iger Lösungen von N-Alkylaminomaltiten, daraus hergestellten IEM-Monomeren und Homopolymeren

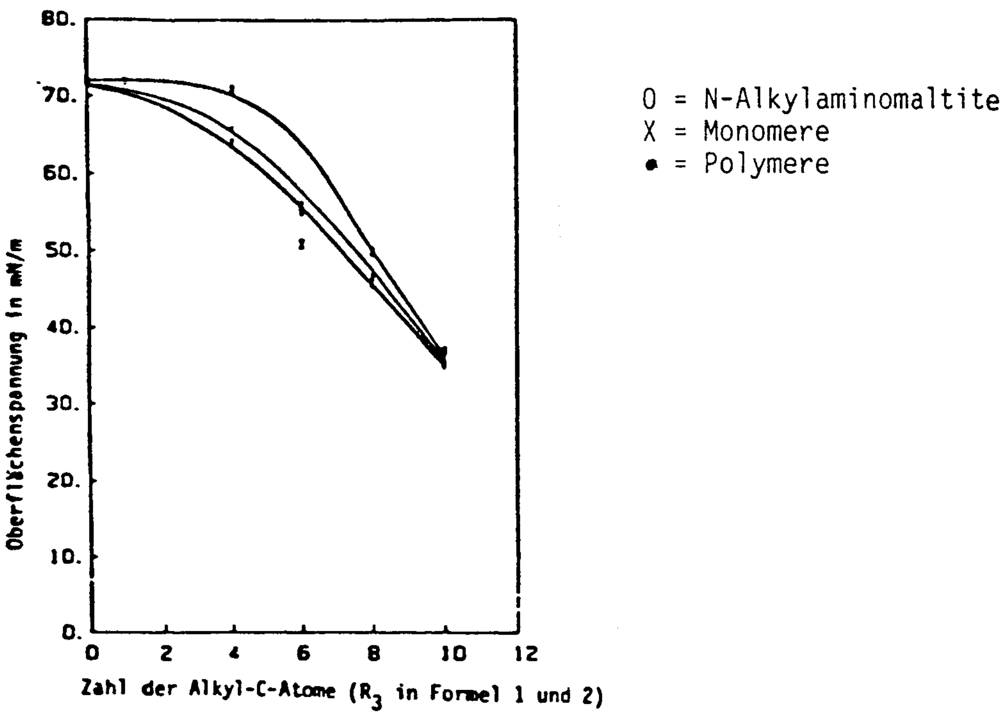


Polymerisationsfähige Monomere auf Basis von Aminopolyolen, die durch reduktive Aminierung aus reduzierenden Zuckern erhalten wurden, sind in neuerer Zeit mehrfach beschrieben (zum Beispiel DE 36 25 931 C2 und J. Klein, D. Herzog, Makromol. Chem. 188, 1217-1232 (1987)).

Diese Monomeren sind nicht oberflächenaktiv (zum Beispiel Abb. 1, $R_3$ = 0). Sie eignen sich daher nicht zur Darstellung von oberflächenaktiven Polymeren. Die direkte Umsetzung von N-Alkyl-N-aminopolyolen mit zum Beispiel Methacrylsäure zu den entsprechenden Amiden ist zwar möglich, aber eine Polymerisation zu Homo- oder Copolymeren gelang nicht (J. Klein, S. Engelke: unveröffentlichte Ergebnisse aus dem Institut für Technische Chemie der TU Braunschweig; siehe auch Diplomarbeit S. Engelke, TU Braunschweig, 1985). N-Alkylaminopolyole, hergestellt durch reduktive Aminierung von reduzierenden Kohlenhydraten mit N-Alkylaminen, lassen sich mit substituierten Isocyanaten zu den entsprechenden erfindungsgemäßen Monomeren umsetzen, die sich überraschenderweise problemlos zu hochmolekularen hydrophilen oberflächenaktiven Produkten polymerisieren ließen. Auch Copolymerisation mit hydrophilen Monomeren, beispielsweise Acrylamid, gelingt ohne Schwierigkeiten.

Hydrophile oberflächenaktive Polymere auf Basis von Kohlenhydraten werden üblicherweise bisher durch polymeranaloge Umsetzung beispielsweise von Cellulose erhalten (Ullmanns Encyklopädie der techn. Chemie, 4. Aufl., Bd. 9, S. 199 ff). Deren Oberflächenaktivität ist aber geringer als die der erfindungsgemäßen Monomeren bzw. daraus hergestellten Polymeren bei Verwendung von Alkylketten ($R_3$ in Formeln I und II) mit mehr als 6 Kohlenstoffatomen. Daneben ist eine gleichzeitige Optimierung von für die Anwendung wichtigen Parametern, wie Löslichkeit, Oberflächenaktivität und Viskosität bei einer polymeranalogen Umsetzung schlechter steuerbar als bei einer gezielten Polymerisation. Die Variationsbreite der erfindungsgemäßen Monomeren erlaubt bei geeigneter Wahl der Rohstoffe, zum Beispiel der reduzierenden Kohlenhydrate und/oder der N-Alkylamine, aber auch der substituierten Isocyanate und der Comonomeren, ein weites Spektrum von oberflächenaktiven Polymeren unterschiedlichen HLB-Wertes gezielt herzustellen.

Andere oberflächenaktive Polymere, zum Beispiel Blockcopolymere (sog. Pluronics, Ullmanns Encyklopädie der technischen Chemie, 4. Auflage, Bd. 22, S. 498, 1982) werden nicht durch radikalische Polymerisation gewonnen. Ihre Molekulargewichte sind meistens im Vergleich zu den aus den erfindungsgemäßen Monomeren hergestellten Polymeren bzw. Copolymeren geringer.

In neuerer Zeit wurde über die Synthese von amphiphilen Kohlenhydratpolymeren durch Ringöffnungspolymerisation von octadecylsubstituierten Anhydrozuckern berichtet (Makromol. Chem. 189, 1019-1026, 1988).

Die Darstellung dieser Polymere im technischen Maßstab erscheint wegen der aufwendigen Synthese der substituierten Anhydrozucker eher unwahrscheinlich. Darüber hinaus ist die Übertragung des Syntheseweges auf Oligosaccharide kaum praktikabel.

Wasserlösliche bzw. hydrophile Polymere haben vielseitige Anwendungsmöglichkeiten. Sie werden als Dickungsmittel in der Technik, Pharma- und Nahrungsmittelindustrie, zur Tertiär-Förderung von Erdöl, aber auch als Hilfsmittel zur Textil- und Papierherstellung benötigt (vergleiche zum Beispiel Chemistry and Technology of water-soluble Polymers, edited by C. A. Finch Plenum Press New York 1983, S. 11 ff.). In vielen Bereichen dieser Anwendungen sind unterschiedliche Oberflächenaktivitäten erforderlich. Insbesondere gilt dieses bei der Tertiärerdölförderung, der Papier- und Dispersionsfarbherstellung (Kessel, D. G.: Erdöl, Erdgas, Kohle (1986), 102, S. 504-508). Ein weiteres wichtiges Anwendungsgebiet für oberflächenaktive Substanzen ist die Polymerherstellung durch Emulsionspolymerisation. Mit den erfindungsgemäß hergestellten Monomeren lassen sich stabile Emulsionen darstellen, so daß bei Verwendung der erfindungsgemäßen Monomere in einer Emulsionspolymerisation keine weiteren oberflächenaktiven Substanzen zugesetzt werden müssen.

Aus diesen Gründen sind erfindungsgemäß hergestellte Monomere für die gezielte Darstellung von Polymeren mit einem weiten Anwendungsbereich besonders geeignet.

Die nachstehenden Beispiele dienen zur weiteren Erläuterung der Erfindung, wobei zunächst am Beispiel von N-Alkyl-isomalt die Herstellung von N-Alkyl-saccharidalkoholen gezeigt wird.

Allgemeine Reaktionsbedingungen für die reduktive Aminierung.

| | |
|---|---|
| - Wasserstoffdruck: | 100 - 200 bar |
| - Temperatur: | 45 - 60 °C |
| - Rührgeschwindigkeit: | 500 - 1500 Upm |

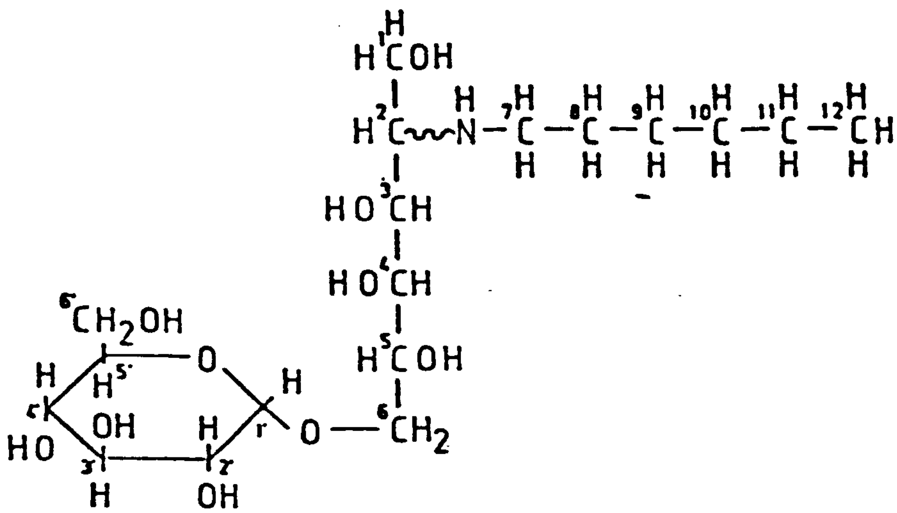

100 g (0,272 mol) Palatinose (Fa. SÜDZUCKER AG) werden in 350 ml destilliertem Wasser und 25 ml Methanol gelöst. Bei 5 °C tropft man 200 ml (1,66 mol) Hexylamin zu und rührt weitere 18 Stunden bei dieser Temperatur.

Die leicht gelbe Lösung wird zusammen mit 25 g wäßriger Raney-Nickel-Suspension (Fa. MERCK) in einen 1-Liter Hochdruckautoklaven eingefüllt. Vor der reduktiven Aminierung wird der Reaktor dreimal mit Wasserstoff (30 bar) gespült und so sauerstofffrei gemacht. Die reduktive Aminierung wird unter den oben

aufgeführten Bedingungen durchgeführt.

Zur Aufarbeitung wird der Autoklav nach 24 Stunden entspannt und entleert. Die wäßrige Phase wird vom Katalysator getrennt und das Amin azeotrop mit dem Wasser am Rotationsverdampfer abgezogen. Durch Zugabe von Wasser und anschließende Etherextraktion werden Aminreste entfernt; eine Gefriertrocknung der wäßrigen Phase liefert das Produkt (Isomerengemisch) in guter Ausbeute.

Beispiele zur Herstellung der erfindungsgemäßen Monomeren

1. Herstellung von N-Ethylmethacrylato-N',N'-decyl,1-desoxymaltit-harnstoff, dem die Formel

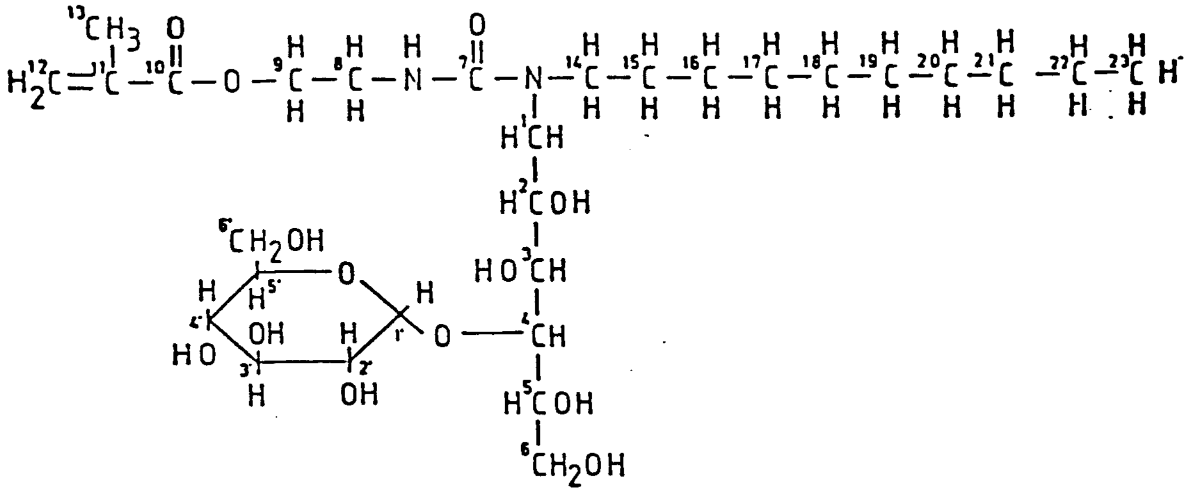

zukommt, in wäßriger Lösung.

Zu einer Suspension von 8 g (16,5 mmol) N-Decylaminomaltit in 40 ml destilliertem Wasser tropft man unter Kühlung bei 5 °C unter starkem Rühren 2,5 g (16,5 mmol) IEM. Nach Beendigung der Zugabe rührt man eine Stunde bei der gleichen Temperatur und weitere zwei Stunden bei 10 °C. Die klare Reaktionslösung wird langsam auf Raumtemperatur erwärmt und dreimal mit peroxidfreiem Ether extrahiert. Das Produkt wird durch schonende Trocknung der wäßrigen Phase isoliert.

2. Herstellung von N-Vinyl-N',N'-hexyl,1-desoxy-maltit-harnstoff, dem die Formel

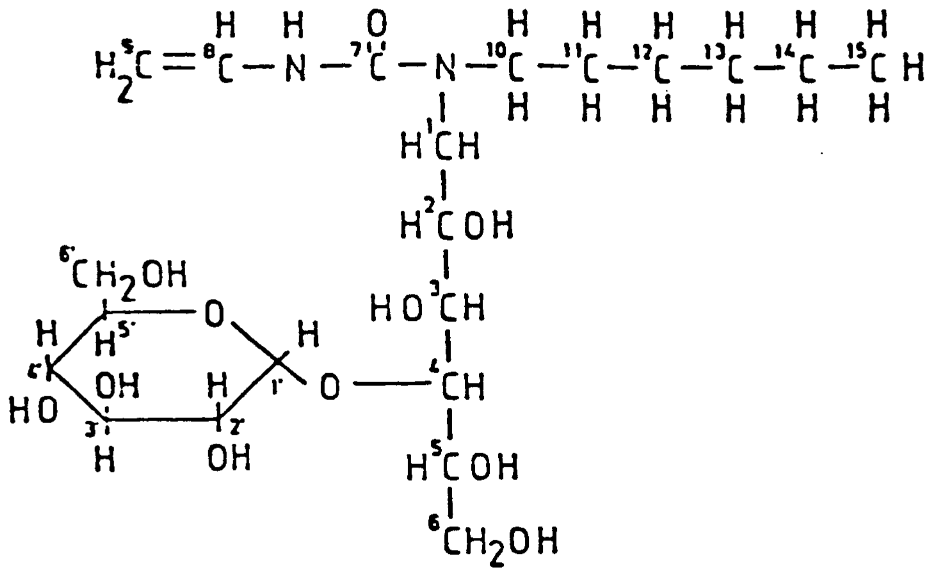

zukommt, in wäßriger Lösung.

5 g (11,7 mmol) N-Hexylaminomaltit werden in 30 ml entionisiertem Wasser suspendiert. Bei einer Temperatur von 10 °C tropft man 0,82 g (11,7 mmol) frischdestilliertes Vinylisocyanat so langsam zu, daß die Temperatur nicht über 12 °C ansteigt. Nach Beendigung der Zugabe wird 12 Stunden bei dieser Temperatur, anschließend zwei Stunden bei 15 °C weitergerührt. Danach läßt man die Reaktionslösung sich auf Raumtemperatur erwärmen und entfernt durch dreimalige Extraktion mit Diethylether unverbrauchtes VIC. Durch Trocknen der wäßrigen Phase erhält man das weiße Produkt.

3. Herstellung von N-Ethylmethacrylato-N',N'-hexyl,2-desoxyisomalt-harnstoff mit der Formel

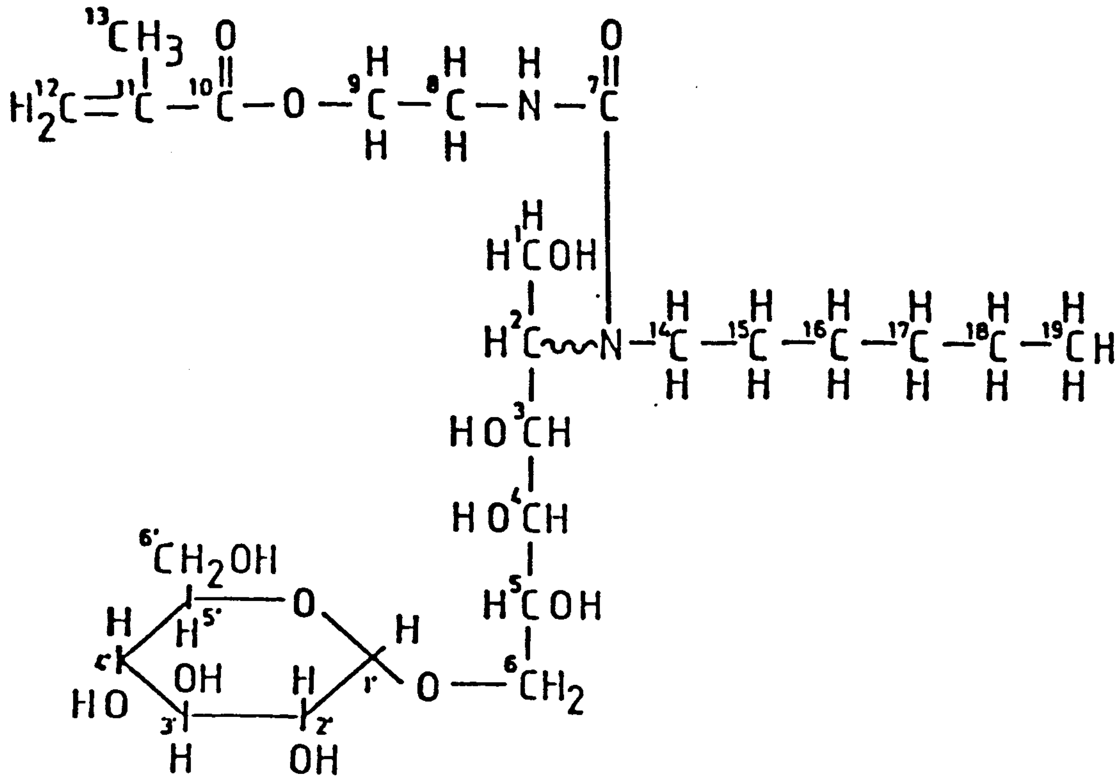

10 g (23,5 mmol) N-Hexylisomalt werden in 50 ml destilliertem Wasser gelöst und auf 0 °C gekühlt. Unter kräftigem Rühren tropft man 3,7 g (23,5 mmol) IEM zu, rührt eine Stunde bei der gleichen Temperatur und weitere zwei Stunden bei +5 °C. Danach läßt man die Lösung sich auf Raumtemperatur erwärmen, extrahiert mit peroxidfreiem Ether, trennt die wässerige Phase ab und isoliert das Produkt durch Gefriertrocknung.

Ausbeute: 89%

| Elementaranalyse | %C | %H | %N |
|---|---|---|---|
| Ber.: | 51,63 | 7,75 | 4,82 |
| Gef.: | 51,55 | 7,83 | 4,87 |

4. Herstellung von N-Ethylmethacrylato-N',N'-octyl,1-desoxy-maltit-harnstoff, dem die Formel

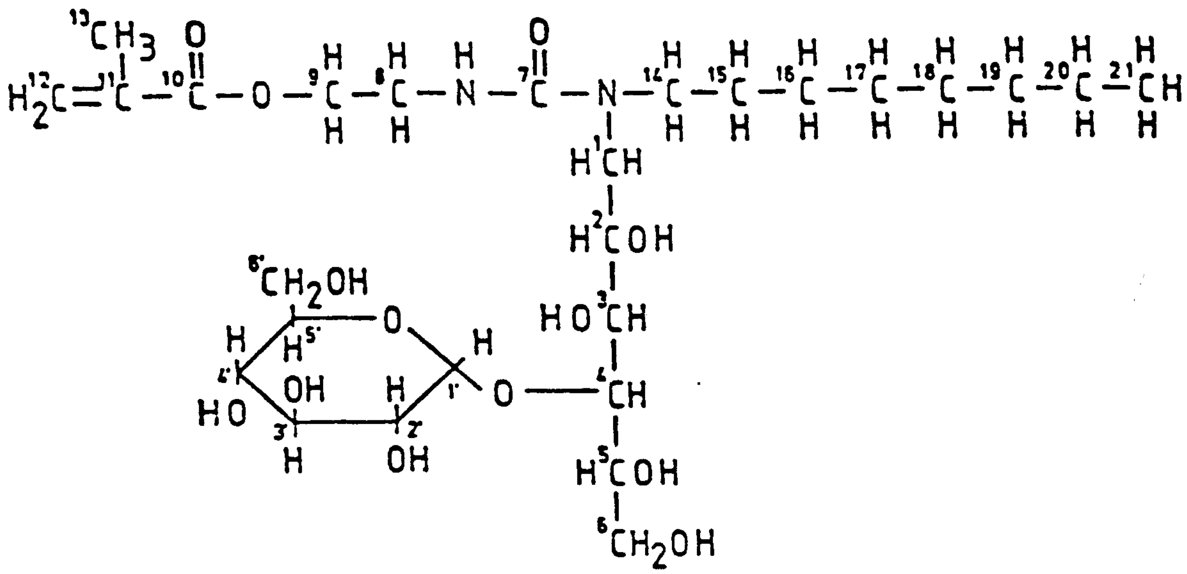

zukommt, in Dioxan.

In einem thermostatisierbaren 150 ml-Reaktor mit Magnetrührer und Innenthermometer werden 6,2 g (13,6 mmol) N-Octylaminomaltit in 50 ml Dioxan suspendiert und bei 15 ° C tropfenweise mit 2,11 g (13,6 mmol) IEM versetzt. Nach vollständiger Zugabe des Isocyanates rührt man eine Stunde bei dieser Temperatur weiter und zur Vervollständigung der Reaktion noch weitere 16 Stunden bei 25 ° C. Mit fortschreitender Reaktionsdauer geht die Suspension in eine homogene Lösung über. Die Isolierung des Produktes erfolgt durch Ausfällung in 600 ml Diethylether. Um die Fällung zu vervollständigen, wird die Lösung 24 Stunden bei -30 ° C aufbewahrt. Anschließende Filtration, dreimaliges Waschen mit Diethylether und Lösungsmittelentfernung an der Hochvakuumanlage liefern das gewünschte weiße Produkt.

5. Im nachstehenden wird die Herstellung von oberflächenaktiven Homopolymeren erläutert.

Durch Variation der Polymerisationsbedingungen (Monomer- und Initiatorkonzentration, Temperatur) wird ein breiter Molekulargewichtsbereich abgedeckt. Die Initiierung erfolgt mit dem wasserlöslichen Redox-System Ammoniumperoxodisulfat in Verbindung mit Natriumbisulfid. Der Vorteil des gewählten Redox-Systemes gegenüber anderen Initiatoren liegt in der Eignung auch bei niedrigen Temperaturen, wodurch auch besonders hohe Polymerisationsgrade erhalten werden können. Bei Verwendung von anderen Peroxid-Initiatoren, wie zum Beispiel $H_2O_2$, erfolgt die Polymerisation bei höheren Temperaturen (70-90 ° C), wobei kürzerkettige Polymere anfallen. Die durchgeführten Polymerisationen erfolgten in bidestilliertem Wasser. Um den Sauerstoff zu entfernen, wird Stickstoff über die Lösung geleitet. Die Rührgeschwindigkeit wird hierbei so hoch gewählt, daß durch Blaseneintrag eine Stickstoffspülung der Monomerlösung gewährleistet ist. Die Polymerisation wurde durch Injektion der in Wasser gelösten Initiatoren bei der gewünschten Temperatur gestartet. Der Abbruch der Polymerisation erfolgt durch Belüften des Reaktors und Zugabe von 5 ml entionisiertem, nicht entgastem Wasser. In den aufgeführten Beispielen wurde die Polymerisation meistens bei niedrigen Umsätzen abgebrochen, um eindeutig charakterisierbare Polymere zu erhalten. Hohe Polymerausbeuten sind, wie Beispiel P 206 zeigt, problemlos zu erreichen. Zur Ausfällung des Polymers wird zur Lösung die 10 - 20-fache Menge Methanol/Aceton (Verhältnis 1:1) zugegeben und das gefällte Polymer abgetrennt.

Um gegebenenfalls nicht umgesetztes Monomer von Polymer abzutrennen, was bei der Polymercharakterisierung und bei der Messung der Oberflächenspannung die Ergebnisse verfälschen könnte, wird die wässerige Polymerlösung dialysiert und durch Gefriertrocknung als watteähnliche Substanz zurückgewonnen.

Die genauen Polymerisationsbedingungen sind den folgenden Tabellen und Erläuterungen zu entnehmen.

Tabelle 3

| Polymerisation von N-Ethylmethacrylato-N',N'-hexyl,1-desoxy-glucit-harnstoff | | | | | | |
|---|---|---|---|---|---|---|
| Pol.Nr. | Mon.konz. Mol/l | Oxid.konz. Mol/l | Red.konz. Mol/l | t h | T °C | Ausb. % |
| P.IB.1 | 0.238 | $5.9 \cdot 10^{-4}$ | $7.1 \cdot 10^{-4}$ | 0.5 | 3 | 14 |
| P.IB.2 | 0.119 | $5.9 \cdot 10^{-4}$ | $7.1 \cdot 10^{-4}$ | 0.5 | 10 | 19 |

Tabelle 4

| Polymerisation von N-Ethylmethacrylato-N',N'-butyl,1-desoxy-maltit-harnstoff | | | | | | |
|---|---|---|---|---|---|---|
| Pol.Nr. | Mon.konz. Mol/l | Oxid.konz. Mol/l | Red.konz. Mol/l | t h | T °C | Ausb. % |
| P 101 | 0.181 | $1.8 \cdot 10^{-3}$ | $2.2 \cdot 10^{-3}$ | 5.0 | 25 | 82 |
| P 102 | 0.181 | $9.0 \cdot 10^{-4}$ | $1.1 \cdot 10^{-3}$ | 4.0 | 4 | 80 |
| P 103 | 0.181 | $4.5 \cdot 10^{-4}$ | $5.4 \cdot 10^{-4}$ | 4.0 | 15 | 88 |
| P 104 | 0.181 | $4.5 \cdot 10^{-4}$ | $5.4 \cdot 10^{-4}$ | 4.0 | 4 | 61 |
| P 105 | 0.226 | $4.1 \cdot 10^{-4}$ | $4.9 \cdot 10^{-4}$ | 1.5 | 3 | 67 |
| P 106 | 0.271 | $4.5 \cdot 10^{-4}$ | $5.4 \cdot 10^{-4}$ | 2.0 | 3 | 57 |

Tabelle 5

| Polymerisation von N-Ethylmethacrylato-N',N'-hexyl,1-desoxy-maltit-harnstoff | | | | | | |
|---|---|---|---|---|---|---|
| Pol.Nr. | Mon.konz. Mol/l | Oxid.konz. Mol/l | Red.konz. Mol/l | t h | T °C | Ausb. % |
| P 201 | 0.129 | $6.6 \cdot 10^{-4}$ | $7.9 \cdot 10^{-4}$ | 1.0 | 12 | 27 |
| P 202 | 0.129 | $6.4 \cdot 10^{-4}$ | $7.7 \cdot 10^{-4}$ | 6.0 | 4 | 26 |
| P 203 | 0.172 | $4.4 \cdot 10^{-4}$ | $5.2 \cdot 10^{-4}$ | 4.0 | 5 | 19 |
| P 204 | 0.172 | $3.9 \cdot 10^{-4}$ | $4.7 \cdot 10^{-4}$ | 1.0 | 5 | 45 |
| P 205 | 0.215 | $3.9 \cdot 10^{-4}$ | $4.7 \cdot 10^{-4}$ | 0.5 | 3 | 42 |
| P 206 | 0.215 | $2.7 \cdot 10^{-4}$ | $3.3 \cdot 10^{-4}$ | 2.0 | 7 | 86 |

Tabelle 6

| Polymerisation von N-Ethylmethacrylato-N',N'-octyl,1-desoxy-maltit-harnstoff | | | | | | |
|---|---|---|---|---|---|---|
| Pol.Nr. | Mon.konz. Mol/l | Oxid.konz. Mol/l | Red.konz. Mol/l | t h | T °C | Ausb. % |
| P 301 | 0.082 | $2.9 \cdot 10^{-4}$ | $3.5 \cdot 10^{-4}$ | 0.5 | 10 | 25 |
| P.302 | 0.082 | $1.6 \cdot 10^{-4}$ | - | 1.0 | 50 | 33 |

Tabelle 7

| Polymerisation von N-Ethylmethacrylato-N',N'-hexyl,2-desoxy-isomalt-harnstoff | | | | | | |
|---|---|---|---|---|---|---|
| Pol.Nr. | Mon.Konz. Mol/l | Oxid.Konz. Mol/l | Red.Konz. Mol/l | t h | T °C | Ausb. % |
| P 501 | 0.129 | $6.6 \cdot 10^{-4}$ | $7.9 \cdot 10^{-4}$ | 2 | 10 | 48 |

Aufgrund der schlechten Wasserlöslichkeit des N-Octyl- und N-Decylmonomers (Tabelle 6) wurden diese Polymere in dem Lösungsmittelsystem Wasser/Methanol (Verhältnis 2:1) hergestellt. Polymer P 301

wurde durch Fällungspolymerisation bei 10 °C synthetisiert. Bei Temperaturerhöhung auf 48 - 50 °C ging das Polymer in Lösung. Aus diesem Grunde konnte Polymer P 302 bei 50 °C durch Lösungspolymerisation hergestellt werden. Nach Abziehen des Methanols aus dem Lösungsmittelsystem waren beide Polymere auch bei tieferen Temperaturen wasserlöslich.

6. Im nachstehenden wird die Herstellung von oberflächenaktiven Copolymeren erläutert.

Copolymerisation von N-Ethylmethacrylato-N',N'-octyl-desoxy-maltit-harnstoff mit Acrylamid.

Die Polymerisation erfolgt in gleicher Weise wie in dem zuvor beschriebenen Punkt 5.

Die Monomeren werden trocken eingewogen, in bidestilliertem Wasser gelöst und durch einen Filter in den mit Stickstoff durchspülten Polymerisationsreaktor gegeben. Um den Restsauerstoff zu entfernen, wird weitere vier Stunden Stickstoff über die Lösung geleitet, wobei die Temperatur 5 °C beträgt, um eine Selbstinitiierung zu vermeiden. Erst kurz vor der Initiierung mit Ammoniumperoxodisulfat wird die Temperatur auf 38 °C eingestellt. Die Polymerisationsparameter sind in Tabelle 8 aufgeführt.

Tabelle 8

| Polymerisationsparameter beim AAm/IEM-Octylmaltit-Copolymer (CAOI) | | | | | | |
|---|---|---|---|---|---|---|
| Monomereinwaage | | Monomergesamtkonz. [Mol/l] | Init. [Mol/l] | T °C | t min. | Ausbeute % |
| AAm [g] | Oktylm. [g] | | | | | |
| 1.4 | 0.7 | 0.88 | $8.7 \cdot 10^{-3}$ | 38 | 10 | 34 |

Tabelle 9 zeigt die Zusammensetzung des Monomerengemisches und des Polymeren. Durch $^{13}C$-NMR-Spektroskopie konnte anhand der unterschiedlichen Carbonylsignale der Monomerkomponenten des Polymers über die Zusammensetzung des Copolymers eine Aussage gemacht werden. Es stellte sich heraus, daß das Octylmonomer statistisch in die AAm-Kette eingebaut wird.

Tabelle 9

| Zusammensetzung des Monomergemisches und des Polymeren | | |
|---|---|---|
| Verhältnis | AAm | Octylmonomer |
| Gewichtsv. der Monomeren in Reaktionslösung | 2 | 1 |
| Molverhältnis der Monomere in Reaktionslösung | 18 | 1 |
| Molverhältnis der Monomere im Polymer | 19 | 1 |

Die Eigenschaft der Oberflächenaktivität des Octylmonomers wurde auf das Copolymer übertragen. Die Messung nach Dialyse und Gefriertrocknung ergab einen Wert von 53 mN/m (siehe Tabelle 1). Dies zeigt, daß schon geringe Mengen an amphiphilen Monomeren, copolymerisiert mit üblichen nicht oberflächenaktiven Substanzen, zu tensidartigen Polymeren führen.

Tabelle 10 zeigt die molekularen und makroskopischen Eigenschaften des reinen Octylpolymers, des Copolymers CAOI und PAAm.

Tabelle 10

| Vergleich des Copolymers CAOI mit PAAm und dem Octylpolymer | | | | | | | | |
|---|---|---|---|---|---|---|---|---|
| Polymer | Mol% Octylm. | Gew.% Octylm. | $M_W$ g/mol | [η] ml/g | $A_2$ | $R_G$ nm | dn/dc ml/g | o * mN/m |
| Octylp. | 100 | 100 | $0.8 \cdot 10^6$ | 22 | 1.3 | 40 | 0.113 | 47 |
| CAO1 | 5 | 34 | $5.3 \cdot 10^6$ | 270 | 3.7 | 129 | 0.172 | 53 |
| PAAm ** | 0 | 0 | $5.7 \cdot 10^6$ | 821 | 4.6 | 183 | 0.175 | 73 |

* Oberflächenspannung 0.1%iger Lösungen in destilliertem Wasser
** Vergleichssubstanz

7. Copolymerisation des VIC-Hexylmonomers mit 1-Vinyl-2-pyrrolidon (VP)

Vorversuche hatten gezeigt, daß 1-Vinyl-2-pyrrolidon in 40%-iger wäßriger Lösung bei erhöhten Temperaturen (65 °C) polymerisiert und Molekulargewichte > $1 \cdot 10^5$ g/mol erzielt wurden.

Die Polymerisation des Copolymers erfolgte im Bombenrohr bei 67 °C. Die Polymerisationsparameter sind in Tabelle 11 aufgelistet.

Tabelle 11

| Polymerisationsparameter vom VIC-Hexyl/VP-Copolymer | | | | | | |
|---|---|---|---|---|---|---|
| Monomereinwaage | | Monomergesamtkonz. Mol/l | Initi.* Mol/l | T °C | t h | Ausbeute % |
| VIC-Hexylm. [g] | VP [g] | | | | | |
| 3.5 | 1.5 | 3.44 | $1.1 \cdot 10^{-3}$ | 67 | 16 | 25 |

* 2,2'-Azobis-2-amidinopropanhydrochlorid

Die Zusammensetzung der Polymeren wurde wieder über $^{13}C$-NMR-Spektroskopie ermittelt. Tabelle 12 zeigt die Zusammensetzung des Monomerengemisches und die Zusammensetzung des Copolymeren. Auch bei diesem Copolymer wird das oberflächenaktive Saccharidmonomer statistisch in die PVP-Kette eingebaut.

Tabelle 12

Zusammensetzung des Monomergemisches und des Polymeren

| Verhältnis | VP | | VIC-Hexylmonomer | |
|---|---|---|---|---|
| in g | 3.5 | : | 1.5 | |
| Gewichtsverh. | 2.3 | : | 1 | |
| Molverh. | 11 | : | 1 | |
| Molverh. im Polymer | 12 | : | 1 | durch $^{13}C$-NMR |

**Patentansprüche**

1. Polymerisierbare N'-Polyhydroxy-N'-alkylharnstoffderivate entsprechend den Formeln I oder II

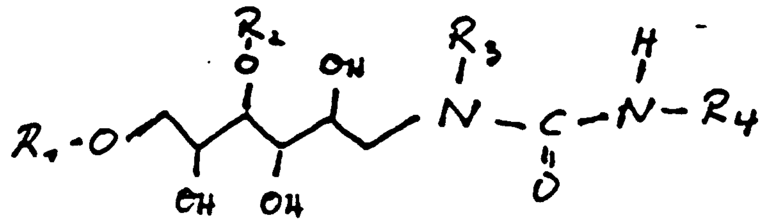

(I)

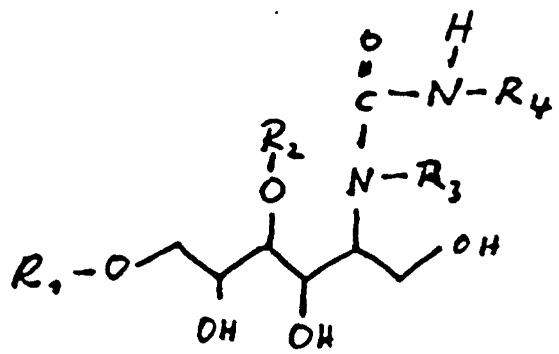

(II)

worin bedeuten:

$R_1$ = H oder ein Mono- oder Oligosaccharid,
$R_2$ = H oder ein Mono- oder Oligosaccharid,
$R_3$ = $C_nH_{2n+1}$ mit n = 4 bis 21, sowie
$R_4$ =
- Alkylmethacrylato-Gruppe,
- Alkylacrylato-Gruppe oder
- Vinyl-Gruppe

und deren Mischungen.

2. Verfahren zur Herstellung der N'-Polyhydroxy-N'-alkylharnstoffderivate gemäß Anspruch 1, dadurch gekennzeichnet, daß man im Anschluß an eine in an sich bekannter Weise erfolgende reduktive Aminierung von reduzierenden Mono- oder Oligosacchariden mittels N-Alkylaminen in Gegenwart von Katalysatoren und Wasserstoff in wäßriger und/oder alkoholischer Lösung bzw. Suspension und Isolierung und Reinigung der N-Alkylaminopolyole diese in an sich bekannter Weise selektiv zu Harnstoffderivaten entsprechend den Formeln I oder II mittels geeignet substituierten Isocyanaten in wäßriger und/oder alkoholischer Lösung oder in inerten aprotisch polaren Lösungsmitteln umsetzt.

3. Verwendung der polymerisierbaren Harnstoffderivate nach Anspruch 1 entsprechend ihren HLB-Werten als Monomere oder Comonomere zur Herstellung von grenzflächenaktiven Homo- oder Copolymeren.

4. Homo- oder Copolymere auf Basis von N'-Polyhydroxy-N'-alkylharnstoffderivaten, erhältlich durch Polymerisation oder Copolymerisation von monomeren Verbindungen der Formeln I und II gemäß Anspruch 1 mittels Redox-Initiation in wässerigem Medium bei Temperaturen zwischen 0 und 100 °C.

## Claims

1. Polymerizable N'-polyhydroxy-N'-alkyl carbamide derivatives corresponding to the formulae I or II

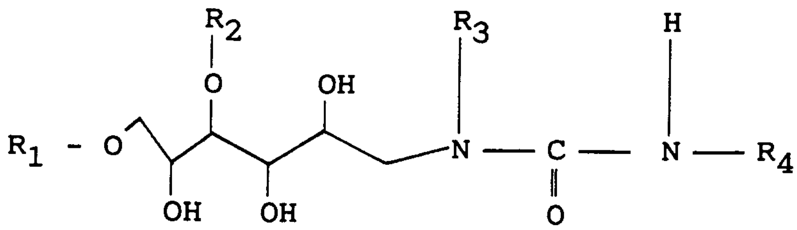

(I)

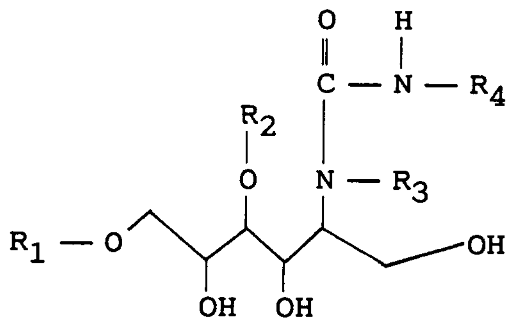

(II)

wherein:

$R_1$ = H or a monosaccharide or oligosaccharide,
$R_2$ = H or a monosaccharide or oligosaccharide,
$R_3$ = $C_nH_{2n+1}$ where n = 4 to 21, and
$R_4$ =

- alkylmethacrylato group,
- alkylacrylato group or
- vinyl group

and their mixtures.

2. Method for producing the N'-polyhydroxy-N'-alkyl carbamide derivatives according to claim 1, characterized in that subsequent to a reductive amination of reducing monosaccharides or oligosaccharides, taking place in a manner known in itself, by means of N-alkylamines in the presence of catalysts and hydrogen in aqueous and/or alcoholic solution or suspension and isolation and purification of the N-alkylaminopolyols, these are caused to react in a manner known in itself selectively to form carbamide derivatives corresponding to the formulae I or II by means of suitably substituted isocyanates in aqueous and/or alcoholic solution or in inert, aprotically polar solvents.

3. Use of the polymerizable carbamide derivatives according to claim 1, corresponding to their HLB values as monomers or comonomers for the production of boundary-active homopolymers or copolymers.

4. Homopolymers or copolymers based on N'-polyhydroxy-N'-alkyl carbamide derivatives, obtainable through polymerization or copolymerization of monomer compounds of the formulae I and II according to claim 1 by means of redox-initiation in aqueous medium at temperatures between 0 and 100 °C.

## Revendications

**1.** Dérivés polymérisables de N'-polyhydroxy-alkylurée selon les formules I ou II

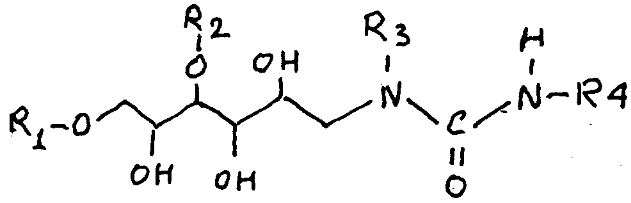

(I)

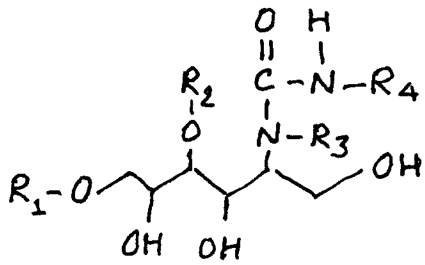

(II)

dans lesquelles :

$R_1$ = H ou un mono- ou oligosaccharide
$R_2$ = H ou un mono- ou oligosaccharide
$R_3$ = $C_nH_{2n+1}$ avec n = 4 à 21, et
$R_4$ =

- groupe alkylméthacrylate
- groupe alkylacrylate
- groupe vinyle

et leurs mélanges.

**2.** Procédé de préparation des dérivés de N'-polyhydroxy-N'alkylurée selon la revendication 1, caractérisé en ce que, à la suite d'une aminisation réductive de mono- ou oligosaccharides au moyen de N-alkylamines en présence de catalyseurs et d'hydrogène en solution aqueuse et/ou alcoolique ou en suspension, et isolement et purification des N-alkylaminopolyols, on convertit ceux-ci sélectivement, d'une manière connue en soi, en dérivés de l'urée selon les formules I ou II au moyen d'isocyanates substitués appropriés en solution aqueuse et/ou alcoolique ou dans des solvants inertes aprotiques polaires.

**3.** Utilisation des dérivés polymérisables de l'urée, selon la revendication 1, en fonction de leurs valeurs HLB, en tant que monomères ou comonomères pour la production de homopolymères ou copolymères tensio-actifs.

**4.** Homopolymères ou copolymères à base de dérivés de N'polyhydroxy-N'alkylurée, pouvant être obtenus par polymérisation ou copolymérisation de combinaisons monomères des formules I et II selon la revendication 1 par initiation redox en milieu aqueux à des températures entre 0 et 100 °C.